# EUROPEAN PATENT APPLICATION

(11) **EP 1 627 722 A1**
(43) Date of publication of application: **22.02.2006**
(21) Application number: 04019392.2
(22) Date of filing: 16.08.2004
(51) Int. Cl.: B29C 47/02, B29C 47/06

(54) **Extrusion process for coating stents, such a coated stent and a system for treating a vascular condition**

(71) Applicant: Medtronic Vascular, Inc., Santa Rosa, California 95403 (US)
(72) Inventor: Neary, Anthony J., Rosshill Galway (IE)
(74) Representative: Hughes, Andrea Michelle

(57) **Abstract**

The present invention provides a method of coating a stent. A stent framework (430) is provided. A polymeric mixture (442) is injected through at least one inlet port (452) in an extrusion die, and the polymeric mixture is extruded through a shaped orifice (454) onto at least a portion of the stent framework (430) to form a coated stent. A coated stent including an extruded coating (440) disposed on at least a portion of the stent framework and a system for treating a vascular condition are also disclosed.

## Description

### FIELD OF THE INVENTION

This invention relates generally to biomedical stents. More specifically, the invention relates to an extruded coating disposed on an endovascular stent, and methods of coating thereof.

### BACKGROUND OF THE INVENTION

Extrusion processes have long been used to coat numerous items such as pipes, cables and wires with various plastics. Conventional extrusion processes use a chamber to hold the material used for extrusion. Mechanical pressure is applied via a compression ram at one end of the chamber, and the material is forced through a patterned opening at the other end to create an extruded form or a coating over an item. For example, a copper wire can be coated with a process that extrudes a polymer from an extrusion die, drawing the polymer onto the wire in a vacuum environment, and applying the polymer to the wire inside the annular extrudate or melt cone.

Extrusion methods have been used to create coatings, sheaths or tubing for protecting or deploying various medical devices that use wires. One example of an extruded polymeric sheath for coating wires is described in "Co-Extruded, Multi-Lumen Medical Lead," Borgersen et al., U.S. Patent Publication 2002/0183824 issued December 5, 2002. The electrically insulating sheath can be used with, for example, implantable cardiac leads for delivering pacing pulses and defibrillation shocks, or sensing a cardiac electrogram (EGM). The body sheath is co-extruded in a co-extrusion process with materials of differing durometers in differing axial sections thereof to create a unitary body sheath. Another method for extruding material onto a wire is described by Solar and others in "Method of Manufacturing a Guidewire with an Extrusion Jacket", U.S. Patent Publication 2002/0084012 issued July 4, 2002. A corewire is fed into an extrusion device and a material is extruded onto the corewire while a gripping apparatus pulls the corewire through the extrusion device to create a coating or extrusion jacket.

Extruded polymeric tubing has been used for medical grafts. An example of a medical graft that has an additional exterior support structure is disclosed in "Endoluminal Graft with Integral Structural Support and Method for Making Same", Edwin et al., U.S. Patent Number 6,053,943 issued April 25, 2000. The structurally supported tubular graft may include a spiraling beading element that is co-extruded with the support structure. The spiraling support structure, which is on the outside of the graft, allows for the expansion of the graft. Unconnected ends of the support structure may have outwardly protruding barbs that upon expansion of the graft secure the graft within a blood vessel or body lumen. The support structure is designed to constrain the tubing of the graft.

An extruded sheath assembled with an expandable stent may be used to constrain the stent until it has reached its areas of deployment. One such sheath is described in "Methods of Forming a Coating for a Prosthesis", Harish et al., U.S. Patent Publication 2002/0122877 issued September 5, 2002. The sheath can be used to constrain an expandable stent until it has reached its areas of deployment. The method for manufacturing the associated stent assembly includes steps of forming a sheath, placing the sheath upon an implantable device or endoluminal prosthesis, and heating the sheath to coat the device or prosthesis. The coating created from the sheath may be used for the delivery of an active ingredient and may have a selected pattern of interstices for allowing a fluid to seep through the coating in the direction of the pattern created.

There are a number of dipping and spraying methods that have been used to apply coatings to the stent framework. A less common method uses injection molding, one example being described in "Polymer-Coated Stent Structure", Loeffler, U.S. Patent 5,897,911 issued April 27, 1999. An exterior mold around the stent controls the thickness of polymer on the exterior surface of the stent. Alternatively, this method may use a preformed sheath of polymer fitted to the interior of the stent whereby a subsequent application of a polymer coats the exterior of the stent.

Although extrusion methods have been developed to extrude coatings on medical devices such as electrical leads and guidewires, little research has focused on the extrusion of a polymeric or drug-polymer coating onto a stent framework. Recent clinical studies on drug-coated vascular stents indicate much therapeutic benefit with the addition of stent coatings that contain pharmaceutical drugs. These drugs may be released from the coating while in the body, delivering their patent effects at the site where they are most needed. Thus, the localized levels of the medications can be elevated, and therefore potentially more effective than orally- or intravenously-delivered drugs that distribute throughout the body.

It would be desirable to have a process for coating and covering a stent with a wide variety of polymers, drugs, and other types of coating materials. The desired process may not require heating and would therefore have minimal impact on pharmaceutical drugs and compounds incorporated into the stent coating. The desired process would use little, if any, solvent, and reduce the amount of drug wasted during typical dipping and spraying cycles. The process would require fewer undesirable chemicals, and reduce or eliminate drying time needed for evaporation of a solvent. The process would allow large quantities of drugs to be included in the coating, and allow various forms of drugs such as micronized powdered drugs and encapsulated drug microspheres to be included within the coating. The method would provide a well-controlled coating thickness, allow a large percentage of drugs within the coating, require little time for application of the desired coating, and overcome the deficiencies and limitations of other coating methods described above.

### SUMMARY OF THE INVENTION

One aspect of the invention provides a method of coating a stent. A stent framework is provided. A polymeric mixture is injected through at least one inlet port in an extrusion die, and the polymeric mixture is extruded through a shaped orifice of the extrusion die onto at least a portion of the stent framework to form a coated stent.

Another aspect of the invention provides a coated stent including a stent framework and an extruded coating disposed on at least a portion of the stent framework.

Another aspect of the invention provides a system for treating a vascular condition, including a catheter and a drug-polymer coated stent coupled to the catheter. The drug-polymer coated stent includes a stent framework and a drug-polymer coating disposed on the stent framework, wherein the drug-polymer coating is extruded onto at least a portion of the stent framework.

The present invention is illustrated by the accompanying drawings of various embodiments and the detailed description given below. The drawings should not be taken to limit the invention to the specific embodiments, but are for explanation and understanding. The detailed description and drawings are merely illustrative of the invention rather than limiting, the scope of the invention being defined by the appended claims and equivalents thereof. The foregoing aspects and other attendant advantages of the present invention will become more readily appreciated by the detailed description taken in conjunction with the accompanying drawings.

### BRIEF DESCRIPTION OF THE DRAWINGS

Various embodiments of the present invention are illustrated by the accompanying figures, wherein:
**FIG. 1** is an illustration of a system for treating a vascular condition including a drug-polymer coated stent coupled to a catheter, in accordance with one embodiment of the current invention;
**FIG. 2** is a cross-sectional view of a drug-polymer coated stent, in accordance with one embodiment of the current invention;
**FIG. 3** is a cross-sectional view of a coated stent, in accordance with one embodiment of the current invention;
**FIG. 4** is a cross-sectional view of a system for coating a stent, in accordance with one embodiment of the current invention;
**FIG. 5** is a cross-sectional view of a system for coating a stent, in accordance with another embodiment of the current invention;
**FIG. 6** is a cross-sectional view of a system for coating a stent, in accordance with another embodiment of the current invention; and
**FIG. 7** is a flow diagram of a method for coating a stent, in accordance with one embodiment of the current invention.

### DETAILED DESCRIPTION OF THE PRESENTLY PREFERRED EMBODIMENTS

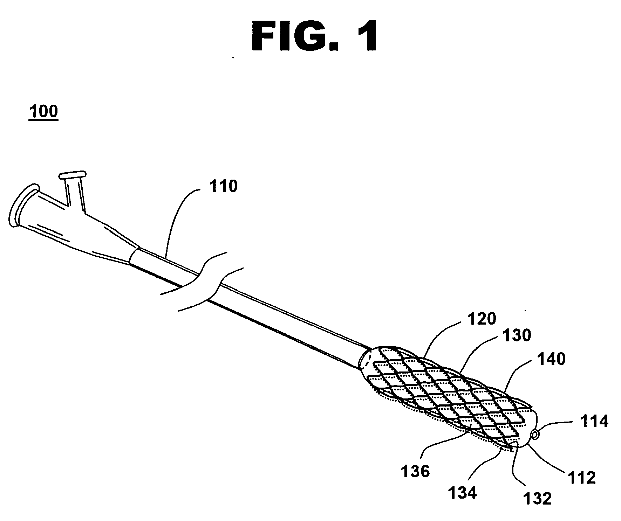

**FIG. 1** shows an illustration of a system for treating a vascular condition, comprising a drug-polymer coated stent coupled to a catheter, in accordance with one embodiment of the present invention at **100.** Coated stent with catheter **100** includes a drug-polymer coated stent **120** coupled to a delivery catheter **110.** Drug-polymer coated stent **120** includes a stent framework **130** and an extruded drug-polymer coating **140** disposed on stent framework **130.** Extruded drug-polymer coating **140** is extruded onto at least a portion of the stent framework, such as an inner surface **132** of stent framework **130,** an outer surface **134** of stent framework **130,** or both inner surface **132** and outer surface **134** of stent framework **130.** Extruded drug-polymer coating **140** comprises a drug-polymer and at least one therapeutic agent. Openings or apertures **136** between struts and elements of stent framework **130** are generally open and free of drug-polymer, though in some cases, drug-polymer coating **140** covers apertures **136,** forming what is sometimes referred to as a covered stent.

Insertion of coated stent **120** into a vessel of a body helps treat, for example, heart disease, various cardiovascular ailments, and other vascular conditions. Catheter-deployed coated stent **120** typically is used to treat one or more blockages, occlusions, stenoses or diseased regions in the coronary artery, femoral artery, peripheral arteries, and other arteries in the body. Treatment of vascular conditions may include the prevention or correction of various ailments and deficiencies associated with the cardiovascular system, the cerebrovascular system, urinogenital systems, biliary conduits, abdominal passageways and other biological vessels within the body.

An exemplary drug-polymer coating **140** includes or encapsulates one or more therapeutic agents. Extruded drug-polymer coating **140** may comprise one or more therapeutic agents dispersed within or encased by drug-polymer layers on coated stent **120,** which are eluted from coated stent **120** with controlled time delivery after the deployment of coated stent **120** into the body. A therapeutic agent is capable of producing a beneficial effect against one or more conditions including coronary restenosis, cardiovascular restenosis, angiographic restenosis, arteriosclerosis, hyperplasia, and other diseases or conditions. For example, the therapeutic agent can be selected to inhibit or prevent vascular restenosis, a condition corresponding to a narrowing or constriction of the diameter of the bodily lumen where the stent is placed. Extruded drug-polymer coating **140** may comprise, for example, an antirestenotic drug such as rapamycin, a rapamycin analogue, or a rapamycin derivative to prevent or reduce the recurrence or narrowing and blockage of the bodily vessel. Drug-polymer coating **140** may comprise an anti-cancer drug such as camptothecin or other topoisomerase inhibitors, an antisense agent, an antineoplastic agent, an antiproliferative agent, an antithrombogenic agent, an anticoagulant, an antiplatelet agent, an antibiotic, an anti-inflammatory agent, a steroid, a gene therapy agent, an organic drug, a pharmaceutical compound, a recombinant DNA product, a recombinant RNA product, a collagen, a collagenic derivative, a protein, a protein analog, a saccharide, a saccharide derivative, a bioactive agent, a pharmaceutical drug, a therapeutic substance, or a combination thereof.

The elution rates of the therapeutic agents and total drug eluted into the body and the tissue bed surrounding the stent framework are based on the thickness of extruded drug-polymer coating **140**, the constituency of drug-polymer coating **140**, the nature and concentration of the therapeutic agents, the thickness and composition of any cap coat, and other factors. Extruded drug-polymer coating **140** may include and elute multiple therapeutic agents to achieve the desired therapeutic effect. Drug-polymer coating **140** can be tailored to control the elution of one or more therapeutic agents, primarily by diffusion processes. In some cases, a portion of drug-polymer coating **140** dissolves and is absorbed into the body, releasing therapeutic agents from within the coating as the therapeutic agents are exposed to the surrounding tissue bed or bodily fluids flowing through the coated stent. In other cases, drug-polymer coating **140** erodes from coated stent **120** to release the therapeutic compounds, the residual polymer being expelled by the body.

Incorporation of a drug or other therapeutic agent into extruded drug-polymer coating **140** allows, for example, the rapid delivery of a pharmacologically active drug or bioactive agent within twenty-four hours following the deployment of a stent, with a slower, steady delivery of a second bioactive agent over the next three to six months. For example, a first therapeutic agent may comprise an antirestenotic drug such as rapamycin, a rapamycin analogue, or a rapamycin derivative. The second therapeutic agent may comprise, for example, an anti-cancer drug such as camptothecin or other topoisomerase inhibitors. The therapeutic agent constituency in the extruded drug-polymer coating may be, for example, between 0.1 percent and 50 percent or more of the drug-polymer coating by weight.

Catheter **110** of an exemplary embodiment of the present invention includes a balloon 112 that expands and deploys the stent within a vessel of the body. After positioning coated stent **120** within the vessel with the assistance of a guide wire traversing through a guidewire lumen **114** inside catheter **110,** balloon **112** is inflated by pressurizing a fluid such as a contrast fluid that fills a tube inside catheter **110** and balloon **112.** Coated stent **120** is expanded until a desired diameter is reached, and then the contrast fluid is depressurized or pumped out, separating balloon **112** from coated stent **120** and leaving coated stent **120** deployed in the vessel of the body. Alternatively, catheter **110** may include a sheath that retracts to allow expansion of a self-expanding version of coated stent **120.**

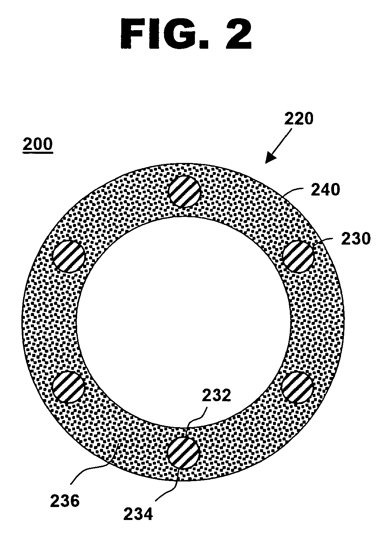

**FIG. 2** shows a cross-sectional view of a drug-polymer coated stent, in accordance with one embodiment of the present invention at **200**. A drug-polymer coated stent **220** includes a stent framework **230** with an extruded drug-polymer coating **240**. In the embodiment illustrated, drug-polymer coating **240** completely jackets or encapsulates an inner surface **232** and an outer surface **234** of stent framework **230**. Apertures **236** between elements of stent framework **230** are filled with drug-polymer coating **240** to form a covered stent. In other embodiments, drug-polymer coating **240** may cover the inner diameter of stent framework **230**, or drug-polymer coating **240** may wrap over outer surface **234** of stent framework **230**. In other embodiments, drug-polymer coating **240** may be subsequently removed from a plurality of apertures **236** within stent framework **230** using, for example, a cutting laser or jets of hot gas.

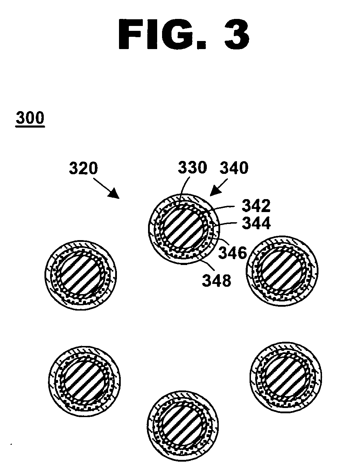

**FIG. 3** shows a cross-sectional view of a coated stent, in accordance with one embodiment of the present invention at **300**. Coated stent **320** includes a stent framework **330** and an extruded stent coating **340** disposed on at least a portion of stent framework **330**. Stent coating **340** includes a drug-polymer **344** with at least one therapeutic agent **346**, and may include a primer coating **342** positioned between drug-polymer **344** and stent framework **330.** Stent coating **340** may include a cap coating **348** positioned on drug-polymer **344**. One or more of primer coating **342**, drug-polymer **344** or cap coating **348** may be extruded onto stent framework **330**.

Stent framework **330** comprises a metallic or polymeric base. Materials such as stainless steel, nitinol, tantalum, MP35N alloy, platinum, titanium, a chromium-based alloy, a cobalt-based alloy, a suitable biocompatible alloy, a suitable biocompatible material, a biocompatible polymer, or a combination thereof are used to form stent framework **330**.

Primer coating **342** is used when needed to improve adhesion between drug-polymer **344** and stent framework **330**, particularly when stent framework **330** comprises a metal such as stainless steel. Primer coating **342** may be applied onto at least a portion of stent framework **330** using application techniques such as dipping, spraying, brushing, painting, or extruding. Primer coating **342** may comprise primer-coating materials such as parylene, polyurethane, phenoxy, epoxy, polyimide, polysulfone, or pellathane.

Drug-polymer **344** comprises an interspersing or encapsulation of at least one polymer and at least one therapeutic agent **346.** Polymers such as poly(vinyl alcohol) (PVA), poly(ethylene-vinyl acetate) (PEVA), polyurethane (PU), polycaprolactone (PCL), polyglycolide (PGA), poly(lactide-co-glycolide) (PLGA), poly(ethylene oxide) (PEO), poly(vinyl pyrrolidone) (PVP), a thermoplastic polymer, a thermoset polymer, a biostable polymer, a biodegradable polymer, a blended polymer, or a combination thereof may be used with one or more therapeutic agents **346** such as camptothecin, rapamycin, a rapamycin analog, or an anti-inflammatant to form drug-polymer **344**. Drug-polymer **344** may be applied onto at least a portion of stent framework **330** using application techniques such as dipping, spraying, brushing, painting, or extruding. Techniques such as dipping, spraying, brushing and painting generally require dissolving or suspending the drug-polymer into a suitable solvent, applying the drug-polymer solution, and drying off the solvent. Extrusion processes allow drug-polymer **344** to be hot formed or cold formed, using polymeric and therapeutic feedstock in various forms such as powders, granules, spheres, pellets, beads, bars, rods, one-part uncured polymers, two-part uncured polymers, and viscous liquids. With extrusion processes, the polymer material may be cross-linked prior to application or after coating, providing additional flexibility in the polymers and therapeutic compounds available for application.

Cap coating **348** can provide protection for underlying drug-polymer **344** or provide an additional barrier for controlling the time-release characteristics of encapsulated therapeutic agents **346**. Cap coating **348** may be applied onto at least a portion of stent framework **330** using application techniques such as dipping, spraying, brushing and painting, or by extruding. Cap coating materials such as polyurethane or polycaprolactone may be applied by extruding.

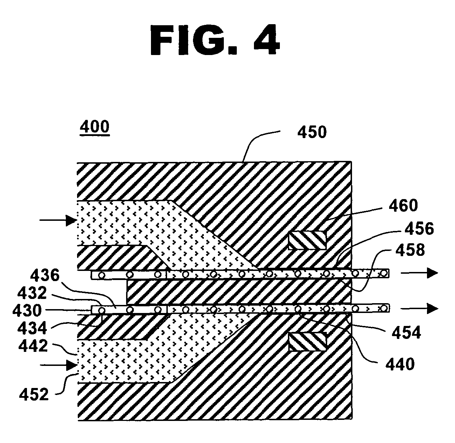

**FIG. 4** shows a cross-sectional view of a system for coating a stent, in accordance with one embodiment of the present invention at **400**. Coating system **400** includes an extrusion die **450** having at least one inlet port **452** and a shaped orifice **454**. A polymeric mixture **442** is injected through inlet port **452**, through shaped orifice **454**, and onto at least a portion of a stent framework **430** to apply a stent coating **440** onto stent framework **430**. In one example, injected polymeric mixture **442** comprises a primer coating material. In another example, injected polymeric mixture **442** comprises a drug-polymer. In another example, injected polymeric mixture **442** comprises a cap coating material.

Polymeric mixture **442** comprises at least one polymer and possibly one or more therapeutic agents. Therapeutic agents in a suitable form such as micronized particles, powder, granules, spheres, pellets or tablets, and the polymer in a suitable form such as powder, granules, spheres, pellets, blocks, rods or billets are combined to form polymeric mixture **442.** Grinding the therapeutic agents and polymer together may help to more evenly distribute the two within polymeric mixture **442**. Compatible solvents may be added to polymeric mixture **442** prior to extrusion for solvating the polymers and therapeutic agents.

The process of extruding stent coating **440** begins with inserting polymeric mixture **442** as feedstock into a commercial extrusion machine, optionally heating polymeric mixture **442,** and then forcing polymeric mixture **442** under pressure onto stent framework **430.** Polymeric mixture **442** is injected into inlet port **452** of extrusion die **450** using any conventional extrusion feed system such as a ram, a screw, or a reciprocating plunger.

Extruded stent coatings **440** are applied to stent framework **430** by passing stent framework **430** through shaped orifice **454** of extrusion die **450** to extrude polymeric mixture **442** onto stent framework **430.** Polymeric mixture **442** may be extruded onto an inner surface **432** of stent framework **430**. Polymeric mixture **442** may be extruded onto an outer surface **434** of stent framework **430**. In the embodiment shown, stent coating **440** is extruded onto stent framework **430**, encapsulating stent framework **430** and forming a covered stent with apertures **436** that are substantially filled with stent coating material.

Shaped orifice **454** may form an annulus, with an outer dimension **456** corresponding to the target outer diameter of stent coating **440,** and an inner dimension **458** corresponding to the target inner diameter of stent coating **440.** A stent framework **430** is loaded into extrusion die **450,** and pushed or pulled through shaped orifice **454** of extrusion die **450** to form an extruded stent coating **440** on stent framework **430.**

An optional heater **460,** which may be integrally formed with extrusion die **450** or wrapped around extrusion die **450,** heats polymeric mixture **442** above an extrusion temperature while polymeric mixture **442** is extruded through shaped orifice **454.**

By using additional extrusion die **450** with different outer dimensions **456** and inner dimensions **458,** various primer coatings, drug-polymers, and cap coatings can be extruded onto stent framework **430** in multiple extrusion sequences, or combined with conventional dipping, brushing, spraying or painting sequences to form the desired coating matrix. Coated stents may undergo further processing steps that remove extruded polymeric mixture **442** from a plurality of apertures within stent framework **430.**

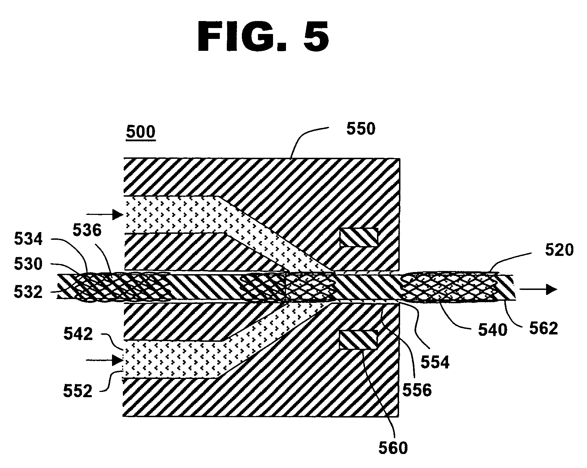

**FIG. 5** shows a cross-sectional view of a system for coating a stent, in accordance with another embodiment of the present invention at **500.** Coating system **500** allows for semi-continuous feeding of stent frameworks to form covered or coated stents **520.** Coating system **500** includes an extrusion die **550** having at least one inlet port **552** and a shaped orifice **554.** A series of stent frameworks **530** may be placed on a mandrel or support wire **562** prior to extruding a polymeric mixture **542.** An inner surface **532** of stent framework **530** contacts an outer surface of mandrel or support wire **562** to allow extrusion of polymeric mixture **542** onto at least an outer surface **534** of stent framework **530.** Outer surface **534** of stent framework **530** is coated.

Polymeric mixture **542** is injected through inlet port **552**, through shaped orifice **554**, and onto at least a portion of a stent framework **530** to apply an extruded stent coating **540** onto stent framework **530**. In one example, injected polymeric mixture **542** comprises a primer coating material. In another example, injected polymer mixture **542** comprises a drug-polymer. In another example, injected polymeric mixture **542** comprises a cap coating material.

Extruded stent coatings **540** are applied to stent framework **530** by passing stent framework **530** through shaped orifice **554** of extrusion die **550** to extrude polymeric mixture **542** onto at least a portion of stent framework **530.** Polymeric mixture **542** may be extruded onto outer surface **534** of stent framework **530,** and may coat individual struts of stent framework **530** or cover stent framework **530** including apertures **536** with stent coating material.

Shaped orifice **554** may form a hole, with a diameter **556** corresponding with the target outer diameter of stent coating **540.** Stent frameworks **530** are loaded onto mandrel or support wire **562,** and pushed or pulled through shaped orifice **554** of extrusion die **550** to form an extruded stent coating **540** on stent framework **530**.

A heater **560** may be integrally formed with extrusion die **550** or wrapped around extrusion die **550** to heat polymeric mixture **542** above an extrusion temperature when polymeric mixture **542** is being extruded through shaped orifice **554**.

Using additional extrusion die **550** with different diameters **556**, primer coatings, drug-polymers, and cap coatings can be extruded onto stent framework **530** with multiple extrusion sequences, or combined with conventional dipping, brushing, spraying or painting sequences to form the desired coating matrix. Additional processing may be performed on coated stents to remove extruded polymeric mixture **542** from a plurality of apertures within stent framework **530.**

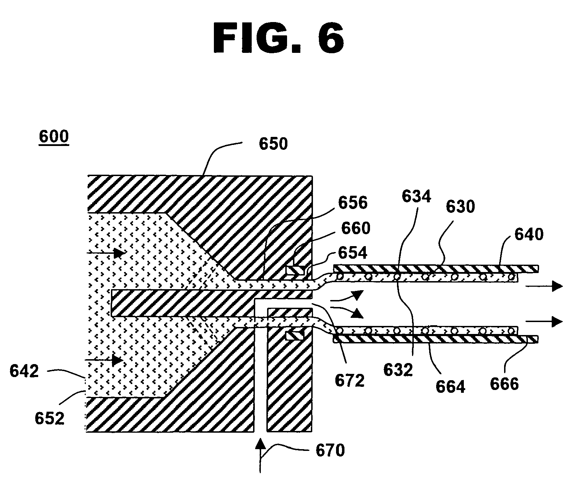

**FIG. 6** shows a cross-sectional view of a system for coating a stent, in accordance with another embodiment of the present invention at **600.** Coating system **600** includes an extrusion die **650** having at least one inlet port **652** and a shaped orifice **654.** A polymeric mixture **642** is injected through inlet port **652,** through shaped orifice **654,** and blown onto at least a portion of an inner surface **632** of a stent framework **630** to apply a stent coating **640** onto stent framework **630.** Shaped orifice **654** typically forms a hole, with a diameter **656** corresponding with the target outer diameter of extruded stent coating **640**. Stent framework **630** is placed adjacent to shaped orifice **654** of extrusion die **650**, and polymeric mixture **642** is extruded through shaped orifice **654** onto at least a portion of stent framework **630**. Stent framework **630** may be placed into a retaining tube **664** prior to extruding polymeric mixture **642**. An outer surface **634** of stent framework **630** contacts an inner surface **666** of retaining tube **664**. Room temperature or hot gas **670** is blown through a gas injector port **672** onto extruded polymeric mixture **642** to force extruded polymeric mixture onto stent framework **630**. Polymeric mixture **642** may be extruded from extrusion die **650**, and blown onto an inner surface **632** of stent framework **630** with gas **670** such as pre-heated nitrogen, argon or air. Alternatively, a vacuum may be applied to force extruded polymeric mixture **642** onto stent framework **630**.

In one example, injected polymeric mixture **642** comprises a primer coating material. In another example, injected polymeric mixture **642** comprises a drug-polymer. In another example, injected polymeric mixture **642** comprises a cap coating material. Multiple passes through coating system **600** may be used to apply multiple coatings or coatings with varying constituency onto at least a portion of stent framework **630.**

Polymeric mixture **642** comprises at least one polymer and may include one or more therapeutic agents. Polymeric mixture **642,** which is inserted as feedstock into a commercial extrusion machine, is optionally heated and forced under pressure to extrude stent coating **640** onto stent framework **630**. Polymeric mixture **642** is injected into inlet port **652** of extrusion die **650** using any conventional extrusion feed system such as a ram, a screw, or a reciprocating plunger. A heater **660** may be integrally formed with extrusion die **650** or wrapped around extrusion die **650** to heat polymeric mixture **642** above an extrusion temperature when polymeric mixture **642** is being extruded through shaped orifice **654.**

With the same or additional extrusion die **650** with a different diameter **656,** various primer coatings, drug-polymers, and cap coatings can be extruded and blown onto stent framework **630** with multiple extrusion sequences, or combined with conventional dipping, brushing, spraying or painting sequences to form the desired coating matrix. Additional processing may be performed on coated stents to remove excess extruded polymeric mixture **642** from a plurality of apertures within stent framework **630**.

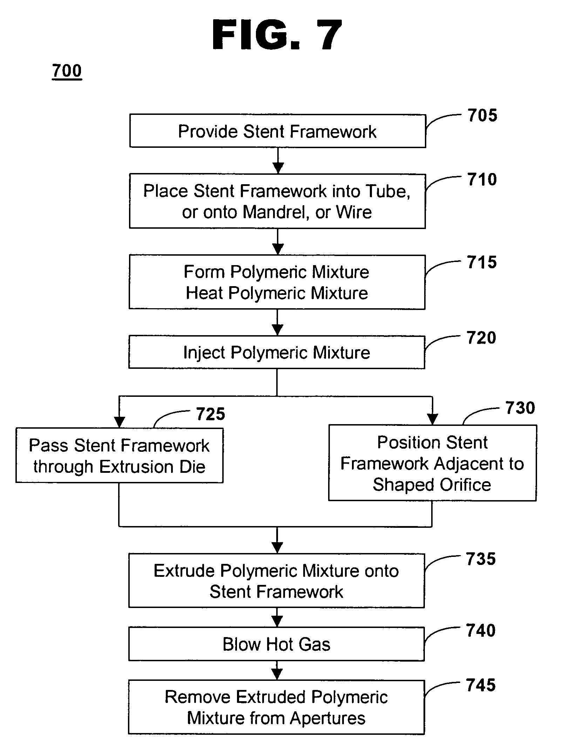

**FIG. 7** shows a flow diagram of a method for coating a stent, in accordance with one embodiment of the current invention at **700**. Stent coating method **700** includes steps to extrude a coating such as a primer coating, a drug-polymer coating, or a cap coating onto a stent.

A stent framework is provided, as seen at block **705**. The stent framework is generally tubular in geometry, with open ends and generally open apertures between struts and stent members that form the stent framework. The stent framework comprises a metallic or polymeric base, including a material such as stainless steel, nitinol, tantalum, MP35N alloy, platinum, titanium, a chromium-based alloy, a cobalt-based alloy, a suitable biocompatible alloy, a suitable biocompatible material, a biocompatible polymer, or a combination thereof. The stent framework may be bare or previously coated, and may be cleaned with various solvents, degreasers and cleansers to remove any debris, residues, or unwanted materials from the surface of the stent framework prior to extruding a coating.

The stent framework may be placed into a retaining tube or onto a mandrel or support wire, as seen at block **710**. Placement of the stent framework into the retaining tube allows an extruded coating to be extruded or blown onto at least an inner surface of the stent framework. Placement of the stent framework onto a mandrel or support wire allows a stent coating to be extruded onto at least an outer surface of the stent framework as the stent framework and mandrel or wire is passed through an extrusion die, and allows the stent frameworks to be individually fed into an extruder or to be continuously or semi-continuously fed into the extruder.

A polymeric mixture including at least one polymer and optionally one or more therapeutic agents is formed and fed into an extruder with the extrusion die, as seen at block **715**. The polymeric mixture may comprise, for example, a primer coating material, a drug-polymer, or a cap coating material. In some cases, a suitable solvent is included in the polymeric mixture. In other cases, the polymeric mixture is solvent-free. The extrusion process allows polymers, drugs, and other therapeutic agents to be hot-formed or cold-formed, using feedstock of various forms such as powders, granules, spheres, pellets, beads, bars, rods and viscous liquids. Typically, the polymeric mixture is extruded without heating to retain the therapeutic agents in a preferred form. The extrusion of coatings at ambient temperatures allows a wide range of polymers and therapeutic agents to be coated onto the stent framework. In some cases, the polymeric mixture may be heated above an extrusion temperature of the polymeric mixture to soften the polymers and to increase the flexibility of the mixture. The polymeric mixture may be pre-heated or heated when the polymeric mixture is extruded through a shaped orifice of the extrusion die with a heater that may be, for example, integrally formed within the extrusion die or wrapped around the extrusion die.

The polymeric mixture is injected through at least one inlet port in the extrusion die, as seen at block **720**. The polymeric mixture is pressurized and extruded through a shaped orifice of the extrusion die onto at least a portion of the stent framework. The shaped orifice may comprise, for example, an annulus with an inner diameter that corresponds to an inner diameter of the coated stent, and a larger outer diameter that corresponds to an outer diameter of the coated stent. In another example, the shaped orifice comprises a hole with a diameter that corresponds to the outer diameter of the coated stent.

In one embodiment, the stent framework is passed through the shaped orifice of the extrusion die, with the polymeric mixture being extruded onto the stent framework to coat or jacket the stent, as seen at block **725**. The stent coating may be formed around the stent framework, encapsulating the stent framework. The polymeric mixture may be extruded onto an inner surface of the stent framework, onto an outer surface of the stent framework, or both. One or more stent frameworks may be placed onto a mandrel or a support wire and fed through the extrusion die to continuously or semi-continuously coat the stents.

In another embodiment, the stent framework is placed into a retaining tube and positioned adjacent to the shaped orifice of the extrusion die, as seen at block **730**. The stent framework may be placed into a retaining tube prior to the polymeric mixture being extruded onto the stent framework.

To form a coated stent, the polymeric mixture is extruded onto at least a portion of the stent framework, as seen at block **735.** The polymeric mixture is extruded through the shaped orifice onto at least a portion of the stent framework.

With the stent framework placed adjacent to the shaped orifice, hot gas such as nitrogen, argon or air may be blown onto the extruded polymeric mixture, forcing the extruded polymeric mixture onto the stent framework, as seen at block **740**. The gas may be at room temperature or may be pre-heated to a temperature typically between room temperature and the glass transition temperature of the polymeric mixture.

The extruded polymer mixture may be removed from a plurality of apertures within the stent framework, as seen at block **745**. Laser cutting, hot gas, and other techniques may be used for removing the extruded coating in selected areas such as the apertures.

Multiple coating steps may be employed to coat a stent, using application techniques such as dipping, spraying, brushing, painting or extruding. Additional extruded coatings may be applied by repeating the aforementioned steps, which place additional coatings such as a cap coat and multiple drug-polymer layers, thereby increasing the quantity of drug delivered to the recipient. Drying steps or baking steps may be interdispersed within the extrusion process steps to provide any additional cross-linking or curing of polymers that may be needed.

When the application of coatings is completed, the coated stent may be coupled to a delivery catheter. For example, the coated stent may be rolled down to compress the stent framework against an inflatable balloon, which is positioned between the coated stent and the catheter for deploying the coated stent in the body. In another example, a self-expanding coated stent may be placed inside a retractable sheath and coupled to the catheter body. Once the stent is inserted and positioned in the body, the sheath can be retracted to deploy the coated stent.

In one exemplary method, a fully processed coated stent is reduced in diameter and placed into the distal end of the catheter to form an interference fit, which secures the stent onto the catheter. The catheter with the stent may be placed in a catheter package and sterilized prior to shipping and storing. Before clinical use, the stent is sterilized using any conventional medically accepted techniques. Sterilization may employ, for example, gamma-ray irradiation, e-beam radiation, ethylene oxide gas, or hydrogen peroxide gas plasma sterilization techniques.

When ready for deployment, the drug-polymer coated stent is inserted into a vessel of the body. The drug-polymer coated stent is inserted typically in a controlled environment such as a catheter lab or hospital. A delivery catheter, which helps position the drug-polymer coated stent in a vessel of the body, is usually inserted through a small incision of the leg and into the femoral artery, and directed through the vascular system to a desired place in the vessel. Guide wires threaded through an inner lumen of the delivery catheter assist in positioning and orienting the drug-polymer coated stent. The position of the drug-polymer coated stent may be monitored, for example, with a fluoroscopic imaging system or an x-ray viewing system in conjunction with radiopaque markers on the coated stent, radiopaque markers on the delivery catheter, or contrast fluid injected into an inner lumen of the delivery catheter and into an inflatable catheter balloon that is coupled to the drug-polymer coated stent. The stent is deployed, for example, by expanding the stent with a balloon or by extracting a sheath that allows a self-expandable stent to enlarge after positioning the stent at a desired location within the body. Prior to deployment, sterilization of the stent using conventional means is completed before clinical use. Once deployed within the body, one or more therapeutic agents that are included or interdispersed within the drug-polymer coating are eluted into the body to deliver their intended therapeutic benefits.

While the embodiments of the invention disclosed herein are presently considered to be preferred, various changes and modifications can be made without departing from the spirit and scope of the invention. The scope of the invention is indicated in the appended claims, and all changes that come within the meaning and range of equivalents are intended to be embraced therein.

## Claims

1. A method of coating a stent, comprising:
providing a stent framework;
injecting a polymeric mixture through at least one inlet port in an extrusion die, the extrusion die having a shaped orifice; and
extruding the polymeric mixture through the shaped orifice onto at least a portion of the stent framework to form a coated stent.

2. The method of claim 1 wherein the injected polymeric mixture comprises a primer coating material.

3. The method of claim 1 wherein the injected polymeric mixture comprises a drug-polymer.

4. The method of claim 1 wherein the injected polymeric mixture comprises a cap coating material.

5. The method of claim 1 wherein the shaped orifice of the extrusion die comprises an annulus, the annulus having an inner diameter corresponding to an inner diameter of the coated stent and an outer diameter corresponding to an outer diameter of the coated stent.

6. The method of claim 1 wherein the polymeric mixture is extruded onto an inner surface of the stent framework.

7. The method of claim 1 wherein the polymeric mixture is extruded onto an outer surface of the stent framework.

8. The method of claim 1 further comprising:
heating the polymeric mixture above an extrusion temperature of the polymeric mixture when the polymeric mixture is extruded through the shaped orifice.

9. The method of claim 1 further comprising:
passing the stent framework through the shaped orifice of the extrusion die to extrude the polymeric mixture onto the stent framework.

10. The method of claim 1 further comprising:
positioning the stent framework adjacent to the shaped orifice of the extrusion die; and
extruding the polymeric mixture through the shaped orifice onto at least a portion of the stent framework.

11. The method of claim 1 further comprising:
blowing hot gas onto the extruded polymeric mixture to force the extruded polymer mixture onto the stent framework.

12. The method of claim 1 further comprising:
removing the extruded polymeric mixture from a plurality of apertures within the stent framework.

13. The method of claim 1 further comprising:
placing the stent framework into a retaining tube prior to extruding the polymeric mixture, wherein an outer surface of the stent framework contacts an inner surface of the retaining tube to allow the polymeric mixture to be extruded onto at least an inner surface of the stent framework.

14. The method of claim 1 further comprising:
placing the stent framework onto one of a mandrel or a support wire prior to extruding the polymeric mixture, wherein an inner surface of the stent framework contacts an outer surface of the mandrel or support wire to allow the polymeric mixture to be extruded onto at least an outer surface of the stent framework.

15. A coated stent comprising:
a stent framework; and
an extruded coating disposed on at least a portion of the stent framework.

16. The stent of claim 15 wherein the stent framework comprises one of a metallic base or a polymeric base.

17. The stent of claim 15 wherein the stent framework comprises a material selected from the group consisting of stainless steel, nitinol, tantalum, MP35N alloy, platinum, titanium, a chromium-based alloy, a cobalt-based alloy, a suitable biocompatible alloy, a suitable biocompatible material, a biocompatible polymer, and a combination thereof.

18. The stent of claim 15 wherein the extruded coating comprises a primer coating.

19. The stent of claim 15 wherein the extruded coating comprises a drug-polymer including at least one therapeutic agent.

20. The stent of claim 19 wherein the therapeutic agent is selected from the group consisting of an antirestenotic drug, an anti-cancer drug, an antisense agent, an antineoplastic agent, an antiproliferative agent, an antithrombogenic agent, an anticoagulant, an antiplatelet agent, an antibiotic, an anti-inflammatory agent, a steroid, a gene therapy agent, a therapeutic substance, an organic drug, a pharmaceutical compound, a recombinant DNA product, a recombinant RNA product, a collagen, a collagenic derivative, a protein, a protein analog, a saccharide, a saccharide derivative, a bioactive agent, a pharmaceutical drug, and a combination thereof.

21. The stent of claim 15 wherein the extruded coating comprises a cap coating.

22. A system for treating a vascular condition, comprising:
a catheter; and
a drug-polymer coated stent coupled to the catheter, the drug-polymer coated stent including a stent framework and a drug-polymer coating disposed on the stent framework, wherein the drug-polymer coating is extruded onto at least a portion of the stent framework.

23. The system of claim 22 wherein the catheter includes a balloon to expand the stent.

24. The system of claim 22 wherein the catheter includes a sheath that retracts to allow expansion of the stent.

25. The system of claim 22 wherein the stent framework comprises one of a metallic base or a polymeric base.

26. The system of claim 22 wherein the stent framework comprises a material selected from the group consisting of stainless steel, nitinol, tantalum, MP35N alloy, platinum, titanium, a chromium-based alloy, a cobalt-based alloy, a suitable biocompatible alloy, a suitable biocompatible material, a biocompatible polymer, and a combination thereof.

27. The system of claim 22 wherein the extruded coating comprises a drug-polymer including at least one therapeutic agent.

28. The system of claim 22 wherein the therapeutic agent is selected from the group consisting of an antirestenotic drug, an anti-cancer drug, an antisense agent, an antineoplastic agent, an antiproliferative agent, an antithrombogenic agent, an anticoagulant, an antiplatelet agent, an antibiotic, an anti-inflammatory agent, a steroid, a gene therapy agent, a therapeutic substance, an organic drug, a pharmaceutical compound, a recombinant DNA product, a recombinant RNA product, a collagen, a collagenic derivative, a protein, a protein analog, a saccharide, a saccharide derivative, a bioactive agent, a pharmaceutical drug, and a combination thereof.
